# FASCICULE DE BREVET EUROPEEN

(11) **EP 0 667 145 B1**
(45) Date de publication et mention de la délivrance du brevet: **25.09.1996**
(21) Numéro de dépôt: 95400062.6
(22) Date de dépôt: 12.01.1995
(51) Int. Cl.: A61K 7/48, A61K 7/06, A61K 31/70

(54) **Composition cosmétique ou dermatologique stabilisée contenant plusieurs précurseurs d'un même actif**
Stabilisierte kosmetische oder dermatologische Zusammensetzung, die mehrere Precursoren des gleichen Wirkstoffs enthaelt
Stabilized cosmetic or dermatologic composition containing several precursors of a same active agent

(30) Priorité: 31.01.1994 FR 9401031
(43) Date de publication de la demande: 16.08.1995
(73) Titulaire: L'OREAL, F-75008 Paris (FR)
(72) Inventeur: Bernard, Dominique, F-75015 Paris (FR); Nguyen, Quang Lan, F-72160 Antony (FR)
(74) Mandataire: Lhoste, Catherine

(56) Documents cités:
- EP-A- 0 371 844
- EP-A- 0 487 404
- EP-A- 0 506 961
- WO-A-86/06275
- FR-A- 2 694 692
- GB-A- 2 266 052
- PATENT ABSTRACTS OF JAPAN vol. 10, no. 350 (C-387) (2406) 26 Novembre 1986 & JP-A-61 152 613 (KANEBO) 11 Juillet 1986
- BERGMEYER, H.U. et GRASSL, M., "Methods of Enzymatic Analysis", 3E edition., vol. IV: Enzymes 2: Esterases, Glycosidases, Lyases, Ligases, pages 230-245, VERLAG CHEMIE, Weinheim, Deerfield Beach, Florida, Basel
- STRYER, L., "Biochemistry", 2e édition, 1981, W.H. FREEMAN AND COMPANY, San Francisco * p. 136

## Description

La présente invention se rapporte à une composition cosmétique ou dermatologique stabilisée contenant plusieurs composés aptes à libérer un même actif, au contact de la peau, y compris le cuir chevelu. Les actifs auxquels s'applique l'invention sont tous les actifs utilisables dans le domaine cosmétique et/ou dermatologique pour lesquels il existe des précurseurs bioconvertibles. Cette composition peut être appliquée sur le visage et/ou le corps humain.

Comme actifs, on peut en particulier cités les vitamines, les lipopeptides, les lipoamino-acides, les α- ou β-hydroxyacides (acide lactique, glycolique, glucuronique), les antioxydants comme les flavonoïdes (quercétine ou rutine, par exemple), les catéchines composant les extraits naturels de plantes telles que le thé, les hydratants comme les polyols (glycérol).

En effet, on cherche, de plus en plus, à introduire dans des compositions cosmétiques et/ou dermatologiques des vitamines comme les vitamines A, B, C, D, E, F ainsi que d'autres actifs en vue d'apporter des traitements spécifiques contre notamment les surcharges pondérales, le vieillissement de la peau, son dessèchement, sa pigmentation, l'acné et certaines maladies de peau (psoriasis), ou encore pour notamment favoriser la cicatrisation et/ou la restructuration de la peau.

En particulier, l'application d'acide ascorbique ou vitamine C sur la peau, en quantité suffisante, permet de stimuler la croissance du tissu conjonctif et notamment celle du collagène. L'acide ascorbique permet également de renforcer les défenses du tissu cutané contre les aggressions extérieures telles que les rayonnements ultra-violets ou la pollution, contre les aggressions des médicaments, de l'alcool, ou du tabac.

Par ailleurs, les tocophérols tels que la vitamine E sont connus pour posséder, à la fois, des propriétés antioxydantes vis-à-vis des phospholipides de la membrane cellulaire, et des propriétés antiradicalaires (ARL) (voir "Radicaux libres et Vitamine E" de J.B. CHAZAN et M.SZULC -Cah. Nutr. Diet. 1987 6 XXII - 1 - pages 66-76).

En outre, la vitamine A ou rétinol, ainsi que les hydroxyacides sont connues pour lutter contre le vieillissement. De plus la vitamine A est connue pour assurer la cicatrisation de la peau.

Malheureusement, la plupart de ces actifs (vitamines, antioxydants, hydroxyacides etc.) est instable en solution, sensible à des facteurs extérieurs rendant ces solutions inopérantes et allant à l'encontre de l'efficacité recherchée.

En particulier, l'article "Stability of ascorbic acid" de BR. HAJRATWALA paru dans "Sciences Pharmaceutiques Revue" pages 281-286, enseigne que l'acide ascorbique possède des propriétés d'instabilité en milieu aqueux, en milieu aérobie et anaérobie, avec une instabilité plus prononcée en milieu aérobie. Il y est illustré notamment le comportement de l'acide ascorbique face à des variations du pH de la solution le contenant, des variations de lumière, de température, face à des composés tels que des tensio-actifs, des solvants, des catalyseurs notamment métalliques.

Aussi, différents moyens ont été envisagés pour stabiliser l'acide ascorbique.

Parmi ces moyens, les brevets japonais JP 89/115 558 et JP 83/129 892 enseignent le blocage du site réactif de l'acide ascorbique, à savoir le site hydroxyle, par estérification et/ou éthérification avec notamment des dérivés phosphatés, sulfatés, alkylés et l'emploi de ces dérivés dans des compositions cosmétiques pour jouer le rôle de la vitamine C.

Malheureusement, ces derniers sont beaucoup moins efficaces que la vitamine C libre (c'est-à-dire sans groupements additionnels).

A cet effet, on a envisagé l'emploi d'un précurseur de la vitamine C.

Ainsi, le brevet européen EP 487 404 divulgue l'utilisation d'un dérivé glucosylé, dans des compositions dermatologiques, apte à libérer de l'acide ascorbique lorsque ces dernières sont mises au contact de la peau.

Par ailleurs, on a envisagé l'estérification d'un dérivé d'acide ascorbique et d'un dérivé de tocophérol avec l'acide phosphorique (voir le document "Bioconversion of a vitamin to vitamins C and E in skin" de KAKUJI TOJO et AE-RIC. LEE publié dans J. Cosmet. Chem., 38, pages 333 - 339) et son utilisation dans une composition.

Mais ce di-ester vis-à-vis de l'acide ascorbique présente une efficacité plus faible que celle de l'acide ascorbique libre et, vis-à-vis de la vitamine E, une activité anti-oxydante moins bonne que celle de la vitamine E libre.

Ce même problème se rencontre aussi pour tout type d'actif.

Il est connu aussi du document EP-A-506961 d'utiliser plusieurs dérivés d'un même actif dans une composition cosmétique, mais ce document n'enseigne pas la coupure enzymatique de ces dérivés sur la peau selon deux mécanismes enzymatiques différents. Par ailleurs, l'efficacité de l'association décrite, ester et phosphate, est encore insuffisante.

Aussi, il subsiste le besoin d'une composition cosmétique et/ou dermatologique contenant des dérivés de vitamine ou de tout autre actif procurant une même efficacité que celles des vitamines ou actifs libres et ainsi qu'une bonne stabilité de ces vitamines ou actifs.

La demanderesse a découvert de façon surprenante que l'utilisation, dans une composition, d'au moins un premier et un second précurseurs d'actif aptes à libérer simultanément un même actif par au moins une première et une seconde réactions enzymatiques spécifiques différentes, le premier précurseur étant choisi parmi les dérivés d'ose d'actif et les amides d'actif et le second précurseur étant choisi parmi les esters et les éthers acylés ou alkylés, permettait de libérer, lors de l'application de la composition sur la peau, une quantité importante d'actif avec une cinétique supérieure à la somme des cinétiques de la première réaction enzymatique et de la seconde réaction enzymatique prises isolément, tout en assurant la stabilité, dans le temps, de l'actif.

Autrement dit, ce couple de précurseurs d'actif, de nature différente, libère, au contact de la peau, l'actif par réaction avec deux systèmes enzymatiques spécifiques différents. En effet, au contact du *Stratum corneum* (couches superficielles de la peau), chaque précurseur d'actif est hydrolysé par l'action d'un enzyme spécifique qui possède sa propre cinétique. L'action simultanée de deux ou plusieurs systèmes enzymatiques différents sur des précurseurs de nature différente d'un même actif, permet de libérer plus rapidement et en plus grande quantité l'actif libre, par comparaison à l'action de systèmes enzymatiques, identiques ou différents, décalée dans le temps.

Cette composition donne de bons résultats, quel que soit le type de peau sur laquelle elle est appliquée, sa température et son taux d'humidité.

Selon l'invention, on peut utiliser deux ou plusieurs précurseurs de nature différente d'un même actif, associés éventuellement à un ou plusieurs autres précurseurs de nature identique ou différente d'un autre actif.

En particulier, le premier et le second précurseurs sont utilisés sous forme de solutions. Le pH de la solution les contenant est de préférence voisin de celui de la surface cutanée, à savoir d'un pH d'environ 5,5, afin d'éviter une quelconque aggression de celle-ci. Si le pH du milieu des deux précurseurs est plus basique ou plus acide que celui de la surface cutanée, alors il est préférable de tamponner ce milieu avec une solution tampon appropriée afin de ramener le pH de ce milieu, proche de 5,5. En pratique, le milieu contenant les deux précurseurs a un pH (typiquement allant de 3,5 à 7,5) compatible avec les activités des enzymes de bioconversion mises en jeu.

Selon l'invention, le premier précurseur est choisi parmi les amides d'actif et les dérivés d'ose d'actif, qui font intervenir respectivement comme enzymes des protéases ou des peptidases et des glycosidases.

Les dérivés d'ose d'actif sont choisis notamment parmi les dérivés osiques en C₃ à C₆. Ils sont choisis notamment parmi les dérivés glucosylés, mannosylés, fructosylés, fucosylés, N-acétylglucosaminés, galactosylés, N-acétyl-galactosaminés, les dérivés de l'acide N-acétylmuramique, les dérivés d'acide sialique et leurs mélanges.

Les amides d'actifs peuvent être par exemple des peptides tels que la lipotyrosine et la trityrosine.

Les seconds précurseurs d'actifs peuvent être choisis parmi les dérivés qui sont hydrolysés par d'autres enzymes, par exemple par des estérases, des phosphatases, des sulfatases, etc. Selon l'invention, on peut choisir par exemple les seconds précurseurs d'actif parmi les phosphates ; les sulfates ; les palmitates, les acétates, les propionates, les férulates et de façon générale les esters alkylés ou acylés d'actif ; les éthers acylés ou alkylés. Les radicaux acylés et alkylés ont en particulier de 1 à 30 atomes de carbone.

En particulier, le second précurseur peut être un ester issu de la réaction avec un acide minéral comme un sulfate ou un phosphate pour réagir avec une sulfatase ou phosphatase au contact de la peau, et le second précurseur un ester acylé ou alkylé issu de la réaction avec un acide organique comme l'acide palmitique, acétique, propionique, nicotinique, 1,2,3 propane tricarboxylique, férulique pour réagir avec une estérase spécifique de la peau.

Quand on utilise plusieurs précurseurs comme seconds précurseurs, ils doivent être choisis de façon à ce que les réactions enzymatiques mises en jeu ne s'inhibent pas. Ainsi, un tartrate d'actif ne peut pas être utilisé en présence d'un phosphate de cet actif du fait que le tartrate est inhibiteur de la phosphatase qui doit hydrolyser le phosphate.

Ainsi, à titre d'exemple pour les vitamines, on peut utiliser un premier précurseur choisi parmi les dérivés d'ose d'une vitamine et un second précurseur choisi parmi les phosphate, sulfate, palmitate, acétate, nicotinate ou propionate de cette vitamine. Ces vitamines sont plus spécialement la vitamine A, la vitamine C et la vitamine E.

Par ailleurs, à titre d'exemple pour l'acide lactique (α-hydroxyacide), on peut utiliser comme premier précurseur un dérivé d'ose d'acide lactique, comme second précurseur le lactate d'éthyle et comme troisième précurseur le dérivé sulfaté de l'acide lactique.

Toujours à titre d'exemple, on peut utiliser comme premier précurseur le glucosyle de quercétine et comme second précurseur un ester de quercétine comme le férulate de quercétine pour libérer de façon synergique la quercétine, lors de l'application sur la peau d'une composition contenant ce couple de précurseurs.

Comme phosphate d'acide ascorbique, on peut utiliser l'ascorbyl phosphate d'un métal alcalin, alcalino terreux ou de transition comme le magnésium, le sodium, le potassium, le calcium, le zinc. Comme phosphate de rétinol, on peut utiliser le rétinyl phosphate d'un métal alcalin ou alcalino terreux comme le magnésium, le potassium.

Comme ester d'acide organique de la vitamine C, on peut utiliser un ester palmitique, acétique ou propionique greffé en position 2 ou 3 de la vitamine C. Comme ester de tocophérol, on peut utiliser les nicotinates ou acétates de tocophérol. Parmi les esters de rétinol, on peut utiliser un ester d'acide palmitique, propionique, acétique.

Parmi les dérivés d'ose de vitamine C utilisables dans l'invention comme premier précurseur, on peut citer à titre d'exemple un dérivé d'ose de vitamine C choisi parmi les dérivés glucosylé, mannosylé, fructosylé, N-acétylglucosaminé, fucosylé, galactosylé, N-acétylgalactosaminé de vitamine C, le dérivé d'acide sialique de vitamine C, le dérivé N-acétylmuramique de vitamine C, ou leurs mélanges.

Pour obtenir une synergie de libération d'un même actif à partir des deux précurseurs, il est préférable d'introduire une quantité de 1 à 40 millimoles de chaque précurseur, et de préférence de 5 à 20 millimoles, la quantité en poids à introduire dépendant du poids moléculaire des dérivés.

En pratique, le premier et le second précurseurs sont présents avantageusement, chacun dans des proportions allant de 0,1 à 10% en poids, et de préférence de 0,5 à 1,5% en poids par rapport au poids total de la composition. La proportion relative entre le premier et le second précurseur peut aller de 10 : 90 à 90 : 10 et est de préférence de 50 : 50 en concentration molaire.

La composition selon l'invention peut se présenter sous forme d'une lotion aqueuse ou hydroalcoolique, d'un gel aqueux ou anhydre, d'un sérum, d'une émulsion huile dans eau (H/E) ou eau dans huile (E/H). Elle peut se présenter aussi sous forme de sphérules comme les liposomes, les nanocapsules, les nanosphères.

Lorsque la composition est une émulsion, la proportion de la phase grasse va de 5 % à 80 % en poids, et est de préférence de 5% à 50% en poids par rapport au poids total de la composition. Les huiles, les émulsionnants et les co-émulsionnants utilisés dans la composition sous forme d'émulsion, sont choisis parmi ceux classiquement utilisés en cosmétique. L'émusionnant et le co-émulsionnant sont présents, dans la composition, en une proportion allant de 0,3% à 30% en poids, et de préférence de 0,5% à 30% en poids par rapport au poids total de la composition.

La composition selon l'invention peut également contenir des additifs cosmétiquement et/ou dermatologiquement acceptables. Ces additifs sont, en particulier, choisis parmi des agents tensio-actifs, des corps gras (huile naturelle, siliconée ou synthétique), des agents hydratants (polyols), des conservateurs, des parfums, des gélifiants (gomme de xanthane, bentone, carbomère), des pigments (TiO₂), des charges (talc) ainsi que des vitamines libres et des filtres UV.

Tous les constituants utilisables dans l'invention doivent en outre être compatibles avec les réactions enzymatiques mises en jeu.

L'invention a encore pour objet une composition stabilisée apte à libérer de la vitamine C, au contact de la peau, caractérisée en ce qu'elle contient au moins un premier précurseur de vitamine C et au moins un second précurseur de cette même vitamine, aptes à libérer simultanément cette vitamine selon respectivement une première et une seconde réactions enzymatiques spécifiques différentes et avec une cinétique supérieure à la somme des cinétiques de la première réaction enzymatique et de la seconde réaction enzymatique prises isolément, et en ce que le premier précurseur est un dérivé d'ose.

Dans un mode préféré de réalisation de la composition de l'invention, on utilise un couple de précurseur de la vitamine C et simultanément de la vitamine E libre ou un de ses dérivés.

Cette composition permet d'obtenir toutes les propriétés de traitement cosmétique ou dermatologique de la vitamine C libre avec une même efficacité que la vitamine libre avec en plus une augmentation de l'activité antioxydante de la vitamine E par rapport à son utilisation seule.

La composition selon l'invention consiste, en particulier en une composition cosmétique et/ou dermatologique pour le traitement cosmétique et/ou dermatologique de la peau. Cette composition permet de lutter par exemple contre le vieillissement cutané, les radicaux libres ou les taches sur la peau, le dessèchement, I'acné et/ou certaines maladies de peau (dermites, psoriasis). Cette composition peut aussi servir pour favoriser la synthèse du collagène ou l'apaisement suite à une activation de certaines enzymes de la peau induites par des stress (oxydatifs ou polluants) ainsi que pour la cicatrisation des plaies.

L'invention a encore pour objet une utilisation de la composition ci-dessus pour le traitement cosmétique de la peau ou pour la préparation d'une crème destinée à un traitement dermatologique.

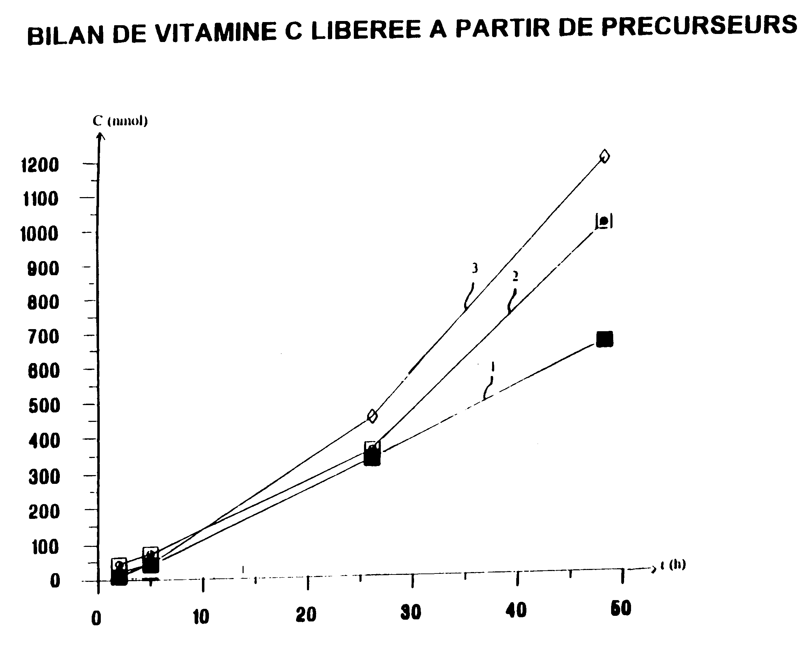

L'invention va maintenant être décrite de façon plus détaillée, à titre illustratif et non limitatif, en référence à l'unique figure annexée montrant l'intérêt d'utiliser un couple de précurseurs de la même vitamine, et aux moyens d'exemples.

Cette figure représente l'évolution de la libération de la vitamine C, pour différents précurseurs au contact du *Stratum corneum*.

L'axe des ordonnées de cette figure représente la quantité (C) de vitamine C libérée, en nanomole, et l'axe des abscisses le temps (t), en heure.

On donne, ci-après, le mode opératoire pour obtenir ces courbes.

Dans un milieu liquide tamponné par un tampon acétate à pH 4, on incorpore de l'ascorbyl phosphate de magnésium, de l'ascorbyl glucosylé et différents échantillons de *Stratum corneum* sous forme de poudre. On mélange l'ensemble, on laisse agir puis on prélève le surnageant. Par un dosage par chromatographie liquide haute performance (HPLC) du surnageant, on obtient les courbes représentées sur la figure.

La courbe 1 a été obtenue pour une concentration nulle en ascorbyl phosphate de magnésium et une concentration en ascorbyl glucosylé 40 millimolaires.

La courbe 2 a été obtenue pour une concentration en ascorbyl phosphate de magnésium 40 millimolaires, et une concentration nulle en ascorbyl glucosylé.

La courbe 3 a été obtenue pour une concentration en ascorbyl phosphate de magnésium 20 millimolaires et une concentration en ascorbyl glucosylé 20 millimolaires.

De cette figure, il ressort clairement une nette amélioration de la libération de l'acide ascorbique à l'état libre au sein du *Stratum corneum* après application sur la peau de la composition selon l'invention (courbe 3) et plus spécialement pour les concentrations élevées de substrats.

On donne ci-après des exemples de compositions conformes à l'invention. Les quantités y sont données en pourcentage pondéral.

### Exemple 1 : Emulsion E/H

### Phase grasse:

| | |
|---|---|
| - Huile naturelle ( beurre de karité) | 20 |
| - Cyclométhicone | 5 |
| - Monostéarate de glycéryle (émulsionnant) | 6 |
| - Huile de vaseline | 7 |

### Phase aqueuse:

| | |
|---|---|
| - Ascorbyl phosphate de magnésium | 1,5 |
| - Ascorbyl glucosylé | 1,5 |
| - Polyol | 3 |
| - Gomme de xanthane | 0,05 |
| - Sulfate de magnésium | 0,4 |
| - Conservateurs et parfums | 1 |
| - Eau qsp | 100 |

L'émulsion se présente sous forme d'une crème blanche destinée au traitement des rides et/ou ridules dûes au vieillissement.

### Exemple 2 :

Cet exemple se différencie de celui de l'exemple 1 par l'utilisation en plus, de 0,5 % de tocophérol. La crème obtenue, présente des propriétés anti-oxydantes améliorées par rapport à celles de l'exemple 1.

### Exemple 3 : Emulsion H/E

| | |
|---|---|
| - Octyl palmitate | 20 |
| - PEG-40 stéarate (émulsionnant) | 2 |
| - Alcool cétylique | 4 |
| - Polyol | 5 |
| - Cyclométhicone | 5 |
| - Vitamine C phosphatée | 1 |
| - Vitamine C glucosylée | 1 |
| - Conservateur | 0,2 |
| - TiO2 (oxyde de titane) | 1 |
| - Parfum | 0,5 |
| - Carbomère (acide polyacrylique) | 0,15 |
| - Eau qsp | 100 |

La crème blanche obtenue est destinée à la protection quotidienne du visage.

## Revendications

1. Composition stabilisée apte à libérer un actif au contact de la peau, caractérisée en ce qu'elle contient au moins un premier et un second précurseurs d'actif aptes à libérer simultanément un même actif par au moins une première et une seconde réactions enzymatiques spécifiques différentes pour libérer une quantité importante d'actif avec une cinétique supérieure à la somme des cinétiques de la première réaction enzymatique et de la seconde réaction enzymatique prises isolément, en ce que le premier précurseur est choisi parmi les dérivés d'ose d'actif et les amides d'actif et en ce que le second précurseur est choisi parmi les esters et les éthers acylés ou alkylés.

2. Composition selon la revendication 1, caractérisée en ce qu'elle est obtenue par mélange d'une première solution contenant le premier précurseur et d'une seconde solution contenant le second précurseur, le mélange des deux solutions ayant un pH voisin de celui de la surface cutanée.

3. Composition selon l'une quelconque des revendications précédentes, caratérisée en ce que les premier et second précuseurs sont choisis parmi les dérivés de vitamine, de lipopeptide, de lipoamino-acide, d' α- ou β-hydroxyacide, d'antioxydant et d'hydratant.

4. Composition selon l'une quelconque des revendications précédentes, caractérisée en ce que le dérivé d'ose est choisi parmi les dérivés glucosylés, mannosylés, fructosylés, fucosylés, N-acétylglucosaminés, galactosylés, N-acétylgalactosaminés, les dérivés de l'acide N-acétylmuramique, les dérivés d'acide sialique et leurs mélanges.

5. Composition selon l'une quelconque des revendications précédentes, caractérisée en ce que le second précurseur est sous forme d'ester.

6. Composition selon l'une quelconque des revendications précédentes, caractérisée en ce que le second précurseur est choisi parmi les esters acylés ou alkylés d'actifs.

7. Composition selon l'une quelconque des revendications 1 à 5, caractérisée en ce que le second précurseur est choisi parmi les esters d'acide minéral ou d'acide organique.

8. Composition selon l'une quelconque des revendications 1 à 5, caractérisée en ce que le premier précurseur est un dérivé d'ose d'actif et le second précurseur est choisi parmi un palmitate, un acétate, un propionate, un nicotinate, un sulfate, un phosphate, un glycéride, un férulate de cet actif.

9. Composition selon la revendication 8, caractérisée en ce que le premier précurseur est choisi parmi les dérivés d'ose de vitamine en C₃ à C₆ ou de quercétine et le second précurseur est choisi parmi les phosphates d'acide ascorbique, les phosphates de rétinol, les nicotinates de tocophérol, les palmitates de rétinol, les palmitates d'acide ascorbique, les acétates de tocophérol, les acétates de rétinol, les acétates d'acide ascorbique, les propionates de rétinol, les propionates d'acide ascorbique, les dérivés de quercétine, les esters de quercétine et leurs mélanges.

10. Composition selon la revendication 9, caractérisée en ce que le dérivé d'ose de vitamine est choisi parmi les dérivés glucosylés, mannosylés, fructosylés, fucosylés, N-acétylglucosaminés, galactosylés, N-acétylgalactosaminés, les dérivés de l'acide N-acétylmuramique, les dérivés d'acide sialique et leurs mélanges.

11. Composition selon l'une quelconque des revendications précédentes, caractérisée en ce que le premier précurseur d'actif et le second précurseur d'actif sont chacun et indépendamment présents dans des proportions allant de 0,1 à 10% en poids par rapport au poids total de la composition.

12. Composition selon l'une quelconque des revendications précédentes, caractérisée en ce que le premier précurseur d'actif et le second précurseur d'actif sont présents chacun dans une proportion molaire de 50/50.

13. Composition selon l'une quelconque des revendications précédentes, caractérisée en ce qu'elle contient en outre au moins un additif choisi parmi les parfums, les agents tensio-actifs, les corps gras, les agents hydratants, les agents conservateurs, les agents gélifiants, les pigments, les charges, les filtres UV.

14. Composition selon l'une quelconque des revendications précédentes, caractérisée en ce qu'elle se présente sous forme d'une émulsion eau dans huile (E/H) ou huile dans eau (H/E).

15. Composition selon la revendication 14, caractérisée en ce que la phase huileuse est choisie dans la gamme allant de 5% à 80% en poids par rapport au poids total de la composition.

16. Composition selon l'une quelconque des revendications précédentes, caractérisée en ce qu'elle consiste en une composition cosmétique et/ou dermatologique.

17. Composition stabilisée apte à libérer de la vitamine C, au contact de la peau, caractérisée en ce qu'elle contient au moins un premier précurseur de vitamine C et au moins un second précurseur de cette même vitamine, aptes à libérer simultanément cette vitamine selon respectivement une première et une seconde réactions enzymatiques spécifiques différentes et avec une cinétique supérieure à la somme des cinétiques de la première réaction enzymatique et de la seconde réaction enzymatique prises isolément, en ce que le premier précurseur est un dérivé d'ose ou un amide et en ce que le second précurseur est choisi parmi les esters et les éthers acylés ou alkylés.

18. Composition selon la revendication 17, caractérisée en ce qu'elle contient de l'ascorbyl phosphate de magnésium et de l'ascorbyl glucosylé.

19. Composition selon l'une des revendications 17 ou 18, caractérisée en ce qu'elle contient également au moins un tocophérol ou l'un de ses dérivés.

20. Utilisation de la composition selon l'une des revendications 1 à 19 pour le traitement cosmétique contre le vieillissement de la peau, les radicaux-libres, sa pigmentation, son déssèchement, l'acné et certaines maladies de la peau ou pour favoriser la synthèse du collagène ou favoriser l'apaisement suite à une activation de certaines enzymes de la peau induite par des stress oxydatifs ou polluants.

21. Utilisation de la composition selon l'une des revendications 1 à 19 pour la préparation d'une crème destinée au traitement dermatologique cicatrisant de plaies.

## Claims

1. Stabilized composition capable of releasing an active agent in contact with the skin, characterized in that it contains at least one first active agent precursor and one second active agent precursor capable of simultaneously releasing the same active agent by at least one first specific enzymatic reaction and one second, different specific enzymatic reaction in order to release a large amount of active agent at a faster rate than the sum of the rates of the first enzymatic reaction and of the second enzymatic reaction taken separately, in that the first precursor is chosen from active agent monosaccharide derivatives and active agent amides, and in that the second precursor is chosen from acyl or alkyl esters and acylated or alkylated ethers.

2. Composition according to Claim 1, characterized in that it is obtained by mixing a first solution containing the first precursor and a second solution containing the second precursor, the mixture of the two solutions having a pH in the vicinity of that of the skin surface.

3. Composition according to either of the preceding claims, characterized in that the first and second precursors are chosen from vitamin, lipopeptide, lipoamino acid, α- or β-hydroxy acid, antioxidant and hydrating agent derivatives.

4. Composition according to any one of the preceding claims, characterized in that the monosaccharide derivative is chosen from glucosyl, mannosyl, fructosyl, fucosyl, N-acetylglucosamine, galactosyl and N-acetylgalactosamine derivatives, N-acetylmuramic acid derivatives, sialic acid derivatives and mixtures thereof.

5. Composition according to any one of the preceding claims, characterized in that the second precursor is in ester form.

6. Composition according to any one of the preceding claims, characterized in that the second precursor is chosen from active agent acyl or alkyl esters.

7. Composition according to any one of Claims 1 to 5, characterized in that the second precursor is chosen from inorganic or organic acid esters.

8. Composition according to any one of Claims 1 to 5, characterized in that the first precursor is an active agent monosaccharide derivative, and the second precursor is chosen from a palmitate, an acetate, a propionate, a nicotinate, a sulphate, a phosphate, a glyceride and a ferulate of this active agent.

9. Composition according to Claim 8, characterized in that the first precursor is chosen from C₃ to C₆ vitamin or quercetin monosaccharide derivatives, and the second precursor is chosen from ascorbic acid phosphates, retinol phosphates, tocopherol nicotinates, retinol palmitates, ascorbic acid palmitates, tocopherol acetates, retinol acetates, ascorbic acid acetates, retinol propionates, ascorbic acid propionates, quercetin derivatives, quercetin esters and mixtures thereof.

10. Composition according to Claim 9, characterized in that the vitamin monosaccharide derivative is chosen from glucosyl, mannosyl, fructosyl, fucosyl, N-acetylglucosamine, galactosyl and N-acetylgalactosamine derivatives, N-acetylmuramic acid derivatives, sialic acid derivatives and mixtures thereof.

11. Composition according to any one of the preceding claims, characterized in that the first active agent precursor and the second active agent precursor are each and independently present in proportions ranging from 0.1 to 10 % by weight relative to the total weight of the composition.

12. Composition according to any one of the preceding claims, characterized in that the first active agent precursor and the second active agent precursor are each present in a molar proportion of 50:50.

13. Composition according to any one of the preceding claims, characterized in that it contains, in addition, at least one additive chosen from perfumes, surfactants, fats, hydrating agents, preservatives, gelling agents, pigments, fillers and UV screening agents.

14. Composition according to any one of the preceding claims, characterized in that it takes the form of a water-in-oil (W/O) or oil-in-water (O/W) emulsion.

15. Composition according to Claim 14, characterized in that the oily phase is chosen to be within the range extending from 5 % to 80 % by weight relative to the total weight of the composition.

16. Composition according to any one of the preceding claims, characterized in that it consists of a cosmetic and/or dermatological composition.

17. Stabilized composition capable of releasing vitamin C in contact with the skin, characterized in that it contains at least one first vitamin C precursor and at least one second precursor of this same vitamin, capable of simultaneously releasing this vitamin according to a first specific enzymatic reaction and a second, different specific enzymatic reaction, respectively, and at a faster rate than the sum of the rates of the first enzymatic reaction and of the second enzymatic reaction taken separately, in that the first precursor is a monosaccharide derivative or an amide, and in that the second precursor is chosen from acyl or alkyl esters and acylated or alkylated ethers.

18. Composition according to Claim 17, characterized in that it contains magnesium ascorbyl phosphate and glucosylated ascorbyl.

19. Composition according to either of Claims 17 and 18, characterized in that it also contains at least one tocopherol or one of its derivatives.

20. Use of the composition according to one of Claims 1 to 19, for cosmetic treatment against skin ageing, free radicals, skin pigmentation, dry skin, acne and certain skin diseases, or to promote collagen synthesis or promote soothing following an activation of certain skin enzymes induced by oxidative or pollution-induced stresses.

21. Use of the composition according to one of Claims 1 to 19, for the preparation of a cream intended for the cicatrizing dermatological treatment of wounds.

## Patentansprüche

1. Stabilisierte Zusammensetzung, die befähigt ist, in Kontakt mit der Haut einen Wirkstoff freizusetzen,
dadurch **gekennzeichnet,** daß
sie mindestens einen ersten und einen zweiten Precursor des Wirkstoffs enthält, die gleichzeitig den gleichen Wirkstoff durch mindestens eine erste spezielle und mindestens eine zweite spezielle, unterschiedliche Enzymreaktion freisetzen können, um so eine wesentliche Menge des Wirkstoffs freizusetzen, wobei die Reaktionsgeschwindigkeit über der Summe der einzeln betrachteten Reaktionsgeschwindigkeiten der ersten und zweiten Enzymreaktion liegt, daß der erste Precursor unter den Zuckerderivaten des Wirkstoffes und den Amiden des Wirkstoffes ausgewählt ist, und dadurch, daß der zweite Precursor unter den Acyl- oder Alkylestern und Acyl- oder Alkylethern des Wirkstoffs ausgewählt ist.

2. Zusammensetzung nach Anspruch 1,
dadurch gekennzeichnet, daß
sie durch Mischen einer ersten Lösung, die den ersten Precursor enthält, und einer zweiten Lösung, die den zweiten Precursor enthält, hergestellt ist, wobei das Gemisch der beiden Lösungen einen pH-Wert in der Nähe des pH-Werts der Hautoberfläche aufweist.

3. Zusammensetzung nach einem der vorhergehenden Ansprüche,
dadurch gekennzeichnet, daß
der erste und der zweite Precursor unter Vitaminderivaten, Lipopeptidderivaten, Lipoaminosäurederivaten, α- oder β-Hydroxysäurederivaten, Derivaten von Antioxidationsmitteln und Derivaten von Hydratisierungsmitteln ausgewählt sind.

4. Zusammensetzung nach einem der vorhergehenden Ansprüche,
dadurch gekennzeichnet, daß
das Zuckerderivat unter den Glucosederivaten, Mannosederivaten, Fructosederivaten, Fucosederivaten, N-Acetylglucosaminderivaten, Galactosederivaten, N-Acylgalactosaminderivaten, den Derivaten von N-Acetylmuraminsäure und den Derivaten der Sialinsäure sowie deren Gemischen ausgewählt ist.

5. Zusammensetzung nach einem der vorhergehenden Ansprüche,
dadurch gekennzeichnet, daß
der zweite Precursor in Form eines Esters vorliegt.

6. Zusammensetzung nach einem der vorhergehenden Ansprüche,
dadurch gekennzeichnet, daß
der zweite Precursor unter Acyl- oder Alkylestern des Wirkstoffs ausgewählt ist.

7. Zusammensetzung nach einem der Ansprüche 1 bis 5,
dadurch gekennzeichnet, daß
der zweite Precursor unter den Estern einer anorganischen oder organischen Säure ausgewählt ist.

8. Zusammensetzung nach einem der Ansprüche 1 bis 5,
dadurch gekennzeichnet, daß
der erste Precursor ein Zuckerderivat des Wirkstoffs ist und der zweite Precursor unter dem Palmitat, Acetat, Propionat, Nicotinat, Sulfat, Phosphat, Glycerid und Ferulat des Wirkstoffs ausgewählt ist.

9. Zusammensetzung nach Anspruch 8,
dadurch gekennzeichnet, daß
der erste Precursor unter den Zuckerderivaten eines Vitamins mit 3 bis 6 Kohlenstoffatomen oder Quercetin und der zweite Precursor unter den Ascorbinsäurephosphaten, Retinolphosphaten, Tocopherolnicotinaten, Retinolpalmitaten, Ascorbinsäurepalmitaten, Tocopherolacetaten, Retinolacetaten, Ascorbinsäureacetaten, Retinolpropionaten, Ascorbinsäurepropionaten, Quercetinderivaten und Quercetinestern sowie deren Gemischen ausgewählt ist.

10. Zusammensetzung nach Anspruch 9,
dadurch gekennzeichnet, daß
das Zuckerderivat eines Vitamins unter den Glucosederivaten, Mannosederivaten, Fructosederivaten, Fucosederivaten, N-Acetylglucosaminderivaten, Galactosederivaten, N-Acetylgalactosaminderivaten, Derivaten von N-Acetylmuraminsäure und Derivaten von Sialinsäure sowie deren Gemischen ausgewählt ist.

11. Zusammensetzung nach einem der vorhergehenden Ansprüche,
dadurch gekennzeichnet, daß
der erste Precursor des Wirkstoffs und der zweite Precursor des Wirkstoffs unabhängig voneinander jeweils in Anteilen im Bereich von 0,1 bis 10 Gew.-%, bezogen auf das Gesamtgewicht der Zusammensetzung, vorliegen.

12. Zusammensetzung nach einem der vorhergehenden Ansprüche,
dadurch gekennzeichnet, daß
der erste Precursor des Wirkstoffs und der zweite Precursor des Wirkstoffs in einem molaren Verhältnis von 50:50 vorliegen.

13. Zusammensetzung nach einem der vorhergehenden Ansprüche,
dadurch gekennzeichnet, daß
sie ferner mindestens einen Zusatzstoff enthält, der unter Parfums, grenzflächenaktiven Stoffen, Fettsubstanzen, Hydratisiserungsmitteln, Konservierungsmitteln, Gelbildnern, Pigmenten, Füllstoffen und UV-Filtern ausgewählt ist.

14. Zusammensetzung nach einem der vorhergehenden Ansprüche,
dadurch gekennzeichnet, daß
sie in Form einer Wasser-in-Öl-Emulsion (W/O) oder einer Öl-in-Wasser-Emulsion (O/W) vorliegt.

15. Zusammensetzung nach Anspruch 14,
dadurch gekennzeichnet, daß
der Anteil der Ölphase im Bereich von 5 bis 80 Gew.-%, bezogen auf das Gesamtgewicht der Zusammensetzung, ausgewählt ist.

16. Zusammensetzung nach einem der vorhergehenden Ansprüche,
dadurch gekennzeichnet, daß
sie eine kosmetische und/oder dermatologische Zusammensetzung ist.

17. Stabilisierte Zusammensetzung, die im Kontakt mit der Haut Vitamin C freisetzen kann,
dadurch gekennzeichnet, daß
sie mindestens einen ersten Precursor von Vitamin C und mindestens einen zweiten Precursor des gleichen Vitamins enthält, die dieses Vitamin gleichzeitig nach einer speziellen ersten bzw. speziellen unterschiedlichen zweiten Enzymreaktion freisetzen können, wobei die Reaktionsgeschwindigkeit über der Summe der einzeln betrachteten Reaktionsgeschwindigkeiten der ersten und zweiten Enzymreaktion liegt, daß der erste Precursor ein Zuckerderivat oder ein Amid ist, sowie dadurch, daß der zweite Precursor unten den Acyl- oder Alkylestern und Acyl- oder Alkylethern ausgewählt ist.

18. Zusammensetzung nach Anspruch 17,
dadurch gekennzeichnet, daß
sie Magnesiumascorbylphosphat und Ascorbylglucose enthält.

19. Zusammensetzung nach einem der Ansprüche 17 oder 18,
dadurch gekennzeichnet, daß
sie ferner mindestens ein Tocopherol oder eines seiner Derivate enthält.

20. Verwendung der Zusammensetzung nach einem der Ansprüche 1 bis 19 zur kosmetischen Behandlung gegen Hautalterung, gegen freie Radikale, gegen Pigmentierung der Haut, gegen Austrocknung der Haut, gegen Akne und verschiedene Hautkrankheiten oder zur Begünstigung der Kollagenbildung oder zur Begünstigung der Linderung nach einer Aktivierung bestimmter Enzyme der Haut, die durch oxidativen Streß oder Verunreinigungen hervorgerufen ist.

21. Verwendung der Zusammensetzung nach einem der Ansprüche 1 bis 19 zur Herstellung einer Creme, die zur dermatologischen Behandlung zur Heilung von Wunden bestimmt ist.
